# EUROPEAN PATENT APPLICATION

(11) **EP 0 861 589 A1**
(43) Date of publication of application: **02.09.1998**
(21) Application number: 97200514.4
(22) Date of filing: 21.02.1997
(51) Int. Cl.: A01H 4/00, C12N 15/82

(54) **Method of generating embryogenic cell cultures for the production of bananas (musa spp.)**

(71) Applicant: K.U. LEUVEN RESEARCH & DEVELOPMENT, 3000 Leuven (BE)
(72) Inventor: Schoofs, Hilde Maria Emmy, 3020 Herent (BE); Sagi, Laszlo, 3001 Heverlee (BE); Panis, Bartholomeus Jozefus, 3010 Kessel-Lo (BE); Remy, Serge, 3910 Neerpelt (BE); Swennen, Rony Leon, 3052 Blanden (BE)
(74) Representative: Van Someren, Petronella F. H. M.

(57) **Abstract**

The present invention relates to a plant regeneration method, comprising the provision of a starting material for regeneration and the regeneration of plants from the starting material, wherein the provision of a starting material comprises proliferating in vitro meristems in the presence of cytokinins in a concentration of at least 50 µM, preferably at least 100µM to obtain cells in suspension. The step of the regeneration of plants in this method is preferably initiated by induction of somatic embryogenesis of cells in suspension on semi-solid medium. The induction step can also be used with other cell suspension preparation methods.

## Description

The present invention relates to a new process for the preparation of regenerable banana embryogenic cell cultures and to their use for long-term storage and for producing optionally transgenic banana plants.

Due to the ease of handling and production seed is by far the preferred vehicle for the propagation and cultivation of most agronomic and forest tree species. However, in some instances uniformly homogeneous seed can either not be produced or utilized for a number of agronomical and horticultural crops. For example, ten out of thirty crops with an annual production of between 10 and 450 million metric tonnes are vegetatively propagated. These crops thus require an alternative vehicle that may be regenerated into a complete plant. Large-scale and uniform multiplication of these crops is achieved by means of biotechnology which is based on a range of in vitro culture techniques including clonal propagation of vegetative meristematic tissues and culture of morphogenic or embryogenic cells and protoplasts.

Bananas are important food crops in the humid and subhumid tropical regions of the world. With an annual production of over 85 million metric tonnes in 1994 bananas are the most important fruit crops worldwide and make a very significant contribution to food security in developing countries. More than 10% of the world's production with a value over US $ 4 billion enters the international export trade and generates an enormous source of income for Third World countries. In the past decades, declining yields or epidemics due to the spread of virulent diseases and pests such as black sigatoka, fusarium wilt and the banana bunchy top disease and nematodes and banana weevils have led to increasing efforts for effective crop protection by using a lot of pesticides. However, not all these pathogens can be treated with chemicals and use of great amounts of pesticides is not only a burden on the environment but also leads to the pests becoming resistant to the pesticide.

Alternatively, several genetic improvement programmes have been initiated to breed disease resistant cultivars. However, bananas are food crops that are exclusively vegetatively propagated and are characterised by a low degree of genetic variability. Conventional breeding of cultivated bananas also remains difficult due to high sterility levels and polyploidy of most edible clones. In addition, only slow progress can be achieved in classical breeding programmes because they must be initiated with poorly characterised and unimproved clones.

Plant tissue culture and molecular genetic techniques have the potential to overcome some of the factors limiting traditional approaches to banana improvement and are thus highly desirable both from an economic and an environmental point of view. These procedures do however depend on successful regeneration of plants from cells or protoplasts.

Somatic embryogenesis is the particular type of differentiation observed from cells or protoplasts. It is the development of embryo-like structures that appear to recapitulate the typical stages in the embryogenesis of a zygote. Plant regeneration methods based on somatic embryos comprise provision of a cell culture, induction of embryogenesis, culturing of embryos and subsequent regeneration and mass clonal propagation of plants. For genetic engineering, the addition of a genetic transformation step before plant regeneration is required. For long-term storage of the cells, protoplasts or embryos cryopreservation methods are useful.

Various prior art plant regeneration methods exist that make use of somatic embryos, either derived from cell cultures or otherwise.

For example, plant regeneration in long-term embryogenic cell suspension cultures as described by Dhed'a et al. (1991) comprises culturing meristematic scalps separated from proliferating shoot-tip cultures in a modified liquid MS medium containing 5 µM 2,4-dichlorophenoxyacetic acid (2,4-D) and 1 µM zeatin.

Other methods are reported on the induction of somatic embryogenesis from alternative explants such as immature zygotic embryos (Cronauer-Mitra and Krikorian, 1988; Escalant and Teisson, 1989), and immature male inflorescences (Escalant et al., 1994).

The average frequency of somatic embryogenesis was in the range of 12-30% and 2-5% for zygotic embryo explants and male inflorescences, respectively, which was efficient enough for the establishment of embryogenic cell suspensions from both types of explants (Marroquin et al., 1993; Cote et al., 1996). A procedure for the induction of cell suspensions from in vitro rhizome and leaf base explants was also described (Novak et al., 1989).

Although the above methods are reproducible in independent laboratories for the generation of regenerable embryogenic banana cell suspensions except for the one described by Novak et al. (1989), each of them has its own shortcomings. A common restriction to these procedures is that they can only be applied to a limited group of bananas. The method of Marroquin et al. (1993) is reported to work with very few, mostly wild species that are able to set seed and thus form zygotic embryos, the explant required for that method. The procedure described by Cote et al. (1996) relies on the availability of male inflorescences which excludes most plantains including the most important cultivars whose male inflorescences are degenerated or not formed at all. In addition, this method requires direct access to the field and the quality of the explants shows seasonal dependence. Finally, though the scalp method as described by Dhed'a et al. (1991) uses the generally and permanently available in vitro shoot-tips as starting material, it has been found to work only for a limited range of cooking bananas and one plantain (Dhed'a, 1992). This method is also characterised by other drawbacks that include low proliferation rates of meristematic explants and blackening of the cultures due to the release of polyphenolic substances. As a result, high quality scalps can not or at best very rarely can be obtained for initiation of somatic embryogenesis. In addition, the method requires prolonged (up to 6 months) culture in liquid medium which increases chances for contamination due to the need for frequent subculturing.

It is the object of the present invention to provide for a general method of plant regeneration from optionally genetically transformed cells, embryos, protoplasts and the like in Cavendish and other types of bananas in which method at least one of the steps of cell culturing, regeneration and cryopreservation identified above is improved as compared to the steps used before this invention.

This is achieved according to the invention by a plant regeneration method, comprising the provision of a starting material for regeneration and the regeneration of plants from the starting material.

The provision of a starting material comprises proliferating in vitro meristems in the presence of cytokinins at a concentration of at least 10 µM, preferably 100 µM. Usually the skilled person would never use this high concentration of cytokinin for proliferation because (s)he would expect it to be phytotoxic.

In another embodiment of the invention the step of the regeneration of plants is initiated by induction of somatic embryogenesis on semi-solid medium. The advantage of a semi-solid induction medium instead of a liquid one is that it is faster, selective, less time- and space-consuming and that there is a lower risk that it will become contaminated. When using a liquid medium for induction, embryogenic cells are diluted and this requires an additional enrichment step which is time-consuming. The step of induction of embryogenesis is not limited to use together with the method described above for provision of starting materials for regeneration in the form of cell suspensions but may also be used with cultures that are provided by any other method.

The meristems are for example shoot tips, but the invention is not restricted to shoot tips.

An optimized proliferation of in vitro meristems prior to scalp preparation and the use of high quality scalps results in embryogenic response in a wide range of banana cultivars. Thus, this procedure eliminates the cultivar-dependent limitations inherent to the published methods as outlined above. In addition, the average frequency of somatic embryogenesis (15-80%) equals or exceeds those obtained with zygotic embryos or with immature male inflorescences.

Furthermore, according to the invention the regeneration of complete plants from optionally transformed cells in culture differs from the regeneration process previously used. The new method is a simplified three-step process in which embryogenic cells are held on semi-solid media resulting in higher plant regeneration frequencies than earlier.

The plant regeneration method of the invention may further comprise a previous or intermediate cryopreservation step either prior to or after the provision of the starting material, which cryopreservation step comprises the addition of sucrose to the material to be cryopreserved in a concentration of at least 0.3 M. The material to be cryopreserved may be the starting material, i.e. the proliferating cultures, of the suspension preparation method. In that case the addition of sucrose thereto is achieved by preculturing the meristems on media containing high sucrose concentrations. This cryoprotective step is followed by rapid freezing. Alternatively, the material to be cryopreserved can be the end-product of this method, i.e. embryogenic cell suspensions, and the addition of sucrose thereto is achieved by a combined treatment with sucrose and dimethylsulfoxide. These cryoprotected suspensions are subjected to slow freezing.

The culturing method of the invention can not only be used to produce embryogenic cell cultures but also somatic embryos and regenerable protoplasts which are suitable for genetic transformation by electroporation and may also be applied to somatic hybridization.

The novel or optimized cryopreservation protocols for long-term storage of both proliferating cultures and embryogenic cell suspensions may also be used in combination with other plant regeneration steps than the ones described here or as such. The invention does not exclude these possibilities. The cryopreservation method is also very suitable for general storage of germplasm.

Optionally either embodiment of the method, namely a method with only the new cell suspension step, or only the induction step or both may further comprise a genetic transformation of the cells before plant regeneration. Thus it becomes possible to genetically modify the resulting regenerated banana plants.

When using the method of the invention it becomes possible to genetically manipulate cells of all major banana groups in the lab by the addition of the genetic transformation step before plant regeneration. A preferred transformation method to be used in the genetic engineering step in the regeneration method of the invention is described by Sági et al. in Bio/Technology 13, 481-485 (1995).

In this application the following definitions apply as explained below:

Bananas: dessert, cooking, highland, beer, ornamental and textile bananas as well as plantains, also commonly described under genus name Musa. Edible bananas are thought to be derived from probably independent inter- and intraspecific hybridization events involving two wild diploid species, M. acuminata and M. balbisiana. The genomic constitution of these two species is designated with AA and BB, respectively. Consequently, depending on how the genomes of these wild species were combined the natural triploid hybrids are characterised by different genomic constitutions. For instance, dessert and highland bananas - mostly AAA, cooking bananas - mostly ABB, plantains - AAB, etc. Approximately 10% of the world banana production enters the international export trade and distributed as dessert banana. By far the most widespread group of dessert bananas is called Cavendish with the commercial clones named Grande Naine and Williams. Plantains are basic food for approximately a hundred million people because they are rich in carbohydrates and can be consumed fresh, cooked or fried. Of the plantains, the cultivars Agbagba/Harton and Dominico Harton are the most important while among cooking bananas the cultivar Bluggoe and Pisang Awak are very common.

Cell and tissue culture: a complex process under sterile or in vitro conditions that comprises the steps of (i) the production of highly proliferating shoot-tip cultures from which embryogenesis-competent scalps are derived, (ii) in vitro culturing of scalps till the formation of embryogenic cultures, and (iii) subsequent maintenance of cell suspensions.

Explant: an organ, a tissue, or cells derived from a plant and cultured in vitro for the purpose of starting a plant cell or tissue culture.

Initiation: the initial cellular proliferation or morphogenic development that eventually results in the establishment of a cell or tissue culture from an explant.

Meristem culture: the in vitro culture of shoot meristems.

Regeneration: a morphogenic response to stimuli that results in the production of organs, embryos, or whole plants from cell or tissue cultures.

Cryopreservation: a method for the long-term storage of cells and tissues, based on the interruption of metabolic activities in cells by the application of ultra-low temperatures, without impairing viability.

Genetic transformation: the unidirectional transfer and incorporation of foreign purified or recombinant DNA into the genome of plant cells. For the production of transgenic plants genetic transformation is an additional step before regeneration.

Protoplast: a plant cell from which the cell wall has been removed.

Somatic embryogenesis: the process of initiation and development of embryos in vitro from somatic cells and tissues.

Embryogenic cells: cells which will develop into somatic embryos upon transfer onto the appropriate regeneration medium.

Embryogenic culture: a plant cell or tissue culture which is capable of forming somatic embryos and regenerating plants via somatic embryogenesis. Embryogenic cultures of bananas consist of a mass of embryogenic cells, early stage somatic embryos and tissues producing such cells and embryos.

Somatic embryo: formed during the process of somatic embryogenesis or after the transfer of embryogenic cells and tissues to media allowing plant regeneration from embryogenic cells.

Early stage somatic embryo: morphologically similar to immature zygotic embryos. In this context, such embryos refer to preglobular, globular embryos and embryos in the early stages of cotyledon formation.

Plantlet: a small plant derived from a germinating somatic embryo.

Rooting: the emergence of the radicle or root from a somatic embryo.

Highly proliferating meristem: in shoot-tip cultures, it implies that high numbers of secondary meristems are formed without any further shoot outgrowth.

Scalp: an explant derived from proliferating shoot-tip cultures by taking the upper 2 to 6 mm layer of a clump of proliferating shoot meristems.

High quality scalp: a scalp prepared from shoot-tip cultures with a cauliflower-like aspect and competent for induction of somatic embryogenesis.

The various steps of the present invention will be further illustrated in the following examples that are in no way intended to limit the invention.

### EXAMPLES

### EXAMPLE 1

### Production of banana highly proliferating meristems from in vitro shoot tips

This example describes an improved method to produce highly proliferating banana in vitro shoot-tip cultures which result in embryogenesis-competent scalps.

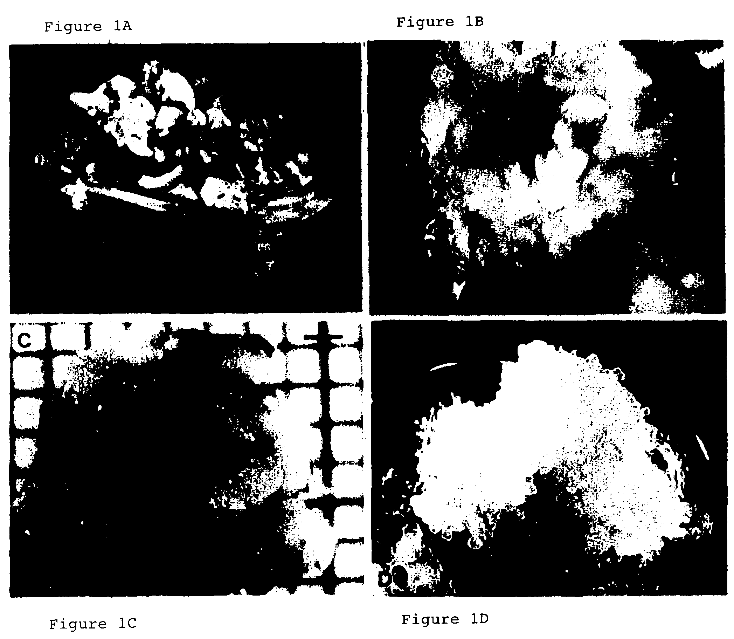

Banana in vitro shoot-tip cultures are started as described by Banerjee et al. (1985). Highly proliferating parts (Figure 1A) are selected from such cultures and transferred to a proliferation medium consisting of a standard basal plant growth medium including but not restricted to that of Schenk and Hildebrandt (1972) or Murashige and Skoog (1962, MS medium) and supplemented with (i) 0.5 µM to 2.5 µM of auxin, preferably IAA and preferably at a concentration of 1 µM, (ii) 50 µM to 250 µM of cytokinin, preferably benzyladenin (BAP) and preferably at a concentration of 100 µM, (iii) 2% to 4% (w/v) of a sugar selected from the group sucrose, glucose, maltose, fructose or combinations thereof, preferably sucrose, at a preferable concentration of 3% (w/v), and solidified with 0.2% (w/v) of Gelrite. This proliferation medium is called p4.

Meristem cultures are grown at about 24°C to about 30°C and preferably at 28°C under fluorescent light at about 20 µeinstein/m²/s to about 80 µeinstein/m²/s of Photosynthetic Active Radiation (PAR) and preferably at 50 µeinstein/m²/s of PAR in standard in vitro culture containers, preferably in 150-ml glass test tubes filled with about 18 ml to about 25 ml of medium p4. The photoperiod of cultures is about 12 h to about 24 h and preferably amounts to 16 h. Under these conditions, the cultures are incubated and subcultured each 1 to 3 months while only highly proliferating parts (see Figure A) are selected for further subculture. The number of subcultures on medium p4 is between of 1 to 10 depending on the cultivar, until proliferating cultures suitable for the induction of somatic embryogenesis are obtained. These proliferating cultures contain many tiny shoot-tips which resemble a 'cauliflower-like' aspect (Figure 1B). Scalps (see example 2) bearing 2 to 20 tiny shoot meristems (Figure 1C) are prepared from such cultures.

A preculture on medium p4 improves in vitro proliferation rates of all cultivars tested so far (Table 1), including but not restricted to:
- Cavendish (AAA) dessert bananas: Williams, Grande Naine, Cavendish 901
- Gros Michel (AAA) dessert bananas: Gros Michel, Highgate
- AAA highland bananas: Igitsiri, Nakitengwa
- AAB dessert bananas: Prata, Lady Finger, Silk
- AAB plantains: Agbagba/Harton, Three Hand Planty, Bise Egome-1, Dominico Harton
- ABB cooking bananas: Bluggoe, Cardaba, Saba, Pisang Awak
- AA edible cultivar: Guyod
- AB edible cultivars: Kamaramasenge, Kisubi
- BB wild diploid: Musa balbisiana Tani

An appropriate preculture with continuous selection of highly proliferating meristems on the proliferation medium p4 is the most critical step to obtain highly proliferating cultures with 'cauliflower-like' aspect (Figure 1B) in a wide range of cultivars. The p4 medium allows for high proliferation rates in up to 10 subculture cycles and the resulting cultures can be directly cryopreserved (example 6). Up to now, no reports suggest the use of such a proliferation medium because one would expect such high concentrations of cytokinins to be toxic for plant cells.

This new method that (i) drastically improves in vitro proliferation rates, (ii) results in embryogenesis-competent explants, and (iii) is less susceptible to tissue blackening has been optimised for banana, but is possibly applicable to the order Zingiberales (e.g. Ensete spp., Heliconia spp., Canna spp.) or other species like palms and cocoyam.

### EXAMPLE 2

### Induction of somatic embryogenesis in banana from scalps

This example shows the induction of embryogenic cultures on semi-solid medium.

Scalps are prepared from optimised in vitro banana meristem cultures (see example 1) by taking the upper 2 mm to 6 mm layer of clumps of 2 to 20 proliferating shoot meristems. Scalps of about 1 mm by about 1 mm to about 7 mm by about 7 mm and preferably 3 mm by 3 mm are induced in standard in vitro culture containers (see example 1) filled with semi-solid induction media consisting of a standard basal plant growth medium, preferably half-strength MS medium supplemented with (i) 4 to 20 µM of auxin, preferably 2,4-D and preferably at a concentration of 5 µM, (ii) 0 to 2 µM of cytokinin, preferably zeatin and preferably at a concentration of 1 µM, (iii) 2% to 6% (w/v) of a sugar selected from the group sucrose, glucose, maltose, fructose or combinations thereof, preferably sucrose, at a preferable concentration of 3% (w/v), and solidified with 0.2% (w/v) of Gelrite. This induction medium is abbreviated as ZZ medium.

Scalp cultures are grown under light and temperature conditions defined in example 1 and maintained without subculturing until the development of an embryogenic culture (Figure 1D) after 5 to 30 weeks of culture depending on the cultivar.

The substitution of semi-solid induction medium for liquid medium induces an embryogenic response in a wide range of banana cultivars including but not restricted to Cavendish dessert banana cultivars (Table 1) and reduces the induction time. The maintenance of scalp cultures without subculturing is more effective compared to frequent refreshments and in addition reduced the risk of contamination.

The scalp-derived embryogenic cultures contain early-stage somatic embryos and embryogenic cells, which can be regenerated as described in example 4. Any other somatic banana explant suitable for induction of somatic embryogenesis might replace the scalp in the method described above.

Scalp-derived embryogenic cultures are induced at a frequency of 15-80% in the following wide range of cultivars:
- Cavendish (AAA) dessert bananas: Williams, Grande Naine, Cavendish 901
- Gros Michel (AAA) dessert bananas: Gros Michel, Highgate
- AAA highland bananas: Igitsiri, Nakitengwa
- AAB dessert bananas: Prata, Lady Finger
- AAB plantains: Agbagba/Harton, Three Hand Planty, Bise Egome-1
- ABB cooking bananas: Bluggoe, Cardaba, Saba
- AA edible cultivar: Guyod
- AB edible cultivars: Kamaramasenge, Kisubi
- BB wild diploid: Musa balbisiana Tani

The list of successful cultivars above indicates that there is no cultivar-dependence and the improved scalp-method is generally applicable in banana. The method could possibly be extended to related species (see example 1).

### EXAMPLE 3

### Production and maintenance of long-term banana embryogenic cell cultures and cell suspensions

This example illustrates the proliferation of embryogenic cultures on semi-solid media and the establishment of embryogenic cell suspensions therefrom in liquid medium.

Prior to transfer to liquid maintenance media, embryogenic cultures are optionally transferred to fresh semi-solid ZZ medium (for composition see example 2) in standard in vitro culture containers preferably Petri dishes or 150-ml glass test tubes filled with 18 ml to 25 ml of ZZ medium. The ZZ medium also allows for a rapid proliferation of embryogenic cell material. Embryogenic banana cultures are subcultured and maintained as such for up to 8 months.

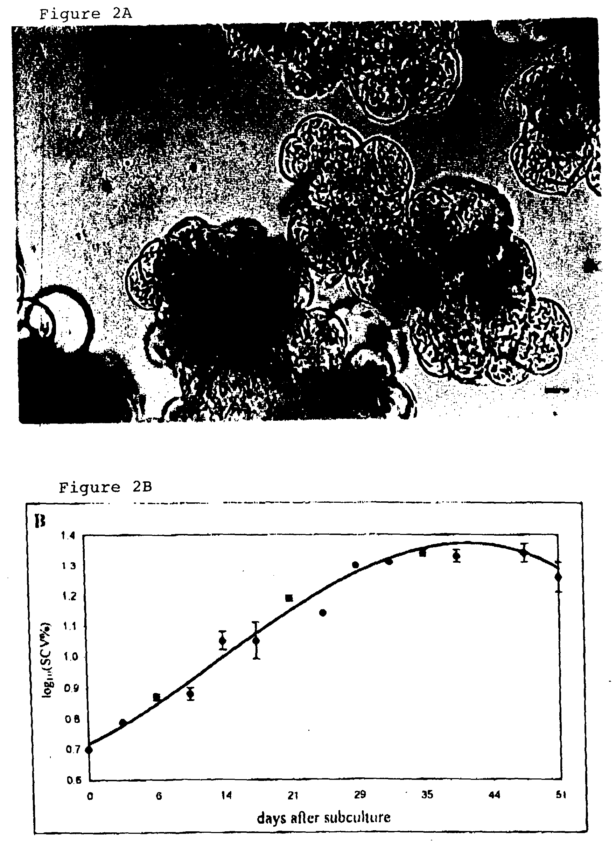

Embryogenic cultures induced as described in example 2 or maintained as above, are transferred to liquid maintenance medium which is identical to the induction medium ZZ (example 2). Per ml of medium 0.02 g to about 0.10 g and preferably 0.04 g fresh weight of embryogenic cell material is incoculated. After 2 to 10 subcultures of each 10 days, embryogenic cell suspensions (Figure 2A) are established with a monthly multiplication rate of 2 to 8 (Figure 2B).

These embryogenic cell suspensions are regenerated to rooted plants in a time span of 3 months to 8 months (example 4). Embryogenic cell suspensions are maintained in Erlenmeyer flasks on a rotary shaker at about 40 rpm to about 120 rpm and preferably at 70 rpm, and grown under the light and temperature conditions defined in example 1. The established embryogenic cell suspensions are subcultured about every 10 days to about every 30 days and preferably about every 14 days.

Scalp-derived regenerable embryogenic cell suspensions are obtained in but are not limited to the following cultivars (Table 1):
- Cavendish (AAA) dessert banana: Williams
- AAA highland bananas: Igitsiri, Nakitengwa
- AAB dessert bananas: Prata
- AAB plantains: Agbagba/Harton, Three Hand Planty, Bise Egome-1
- ABB cooking bananas: Bluggoe, Cardaba, Saba
- AB edible cultivars: Kamaramasenge, Kisubi
- BB wild diploid: Musa balbisiana Tani

Scalp-derived banana embryogenic cell suspensions can be maintained for up to 5 years without loosing their morphogenic potential. These embryogenic cell suspensions are successfully cryopreserved (example 7) and have proven to be most suited to other applications like the isolation of regenerable protoplasts (example 5) and genetic transformation (examples 8 and 9).

**Table 1**

| List of banana cultivars from which meristem cultures with 'cauliflower-like' aspect are obtained. For each of these cultivars it is indicated whether such cultures have resulted in the establishment of embryogenic cultures (EC) or regenerable cell suspensions (ECS). | | | |
|---|---|---|---|
| Group | Cultivar | EC | ECS |
| Cavendish (AAA) dessert bananas | Williams | + | + |
| | Grande Naine | + | - |
| | Cavendish 901 | + | - |
| Gros Michel (AAA) dessert bananas | Gros Michel | + | - |
| | Highgate | + | - |
| AAA highland bananas | Igitsiri | + | + |
| | Nakitengwa | + | + |
| AAB dessert bananas | Prata | + | + |
| | Lady Finger | + | - |
| | Silk | - | - |
| AAB plantains | Agbagba/Harton | + | + |
| | Three Hand Planty | + | + |
| | Bise Egome-1 | + | + |
| | Dominico Harton | ND | ND |
| ABB cooking bananas | Bluggoe | + | + |
| | Cardaba | + | + |
| | Saba | + | + |
| | Pisang Awak | ND | ND |
| AA edible cultivar | Guyod | ND | ND |
| AB edible cultivars | Kamaramasenge | + | + |
| | Kisubi | + | + |
| BB wild diploid | M. balbisiana | + | + |
| + = success - = no success ND = not determined | | | |

### EXAMPLE 4

### Plant regeneration from embryogenic cell cultures of banana

Plant regeneration from banana embryogenic cell suspensions is based on the multistep protocol described by Dhed'a et al. (1991) with modifications as described below.

### Stage 1: The formation of globular to early cotyledonary stage somatic embryos from embryogenic suspension cells

The formation of globular to early cotyledonary stage somatic embryos from embryogenic cells in the suspension cultures is obtained on a standard plant growth medium preferably the standard MS medium supplemented with 2% to 4% (w/v) of a sugar selected from the group sucrose, glucose, maltose, fructose or combinations thereof, preferably sucrose, at a preferable concentration of 3% (w/v), 0 mg/l to 100 mg/l and preferably 100 mg/l of myo-inositol, and 0.2% (w/v) Gelrite. This medium which is not supplemented with growth regulators is called the basal regeneration medium.

Globular to early cotyledonary stage embryos are continuously formed during a period of 1 to 3 months. Prior to plating, the cell suspensions are sieved at 500 µm and washed thoroughly with a liquid hormone-free plant growth medium, preferably the basal regeneration medium as defined above. Per test tube one drop (about 0.05 ml) of a settled cell suspension is added. In Petri dishes 0.05 ml to 0.5 ml of a settled cell suspension are used for regeneration.

### Stage 2: Shoot formation

Stage 1 somatic embryos regenerated from cell suspensions or directly derived from the embryogenic cultures are then transferred individually or in small groups to standard in vitro culture containers and preferably 150-ml glass test tubes filled with the basal regeneration medium supplemented with 0 µM to 10 µM of cytokinin, preferably BAP and preferably at a concentration of 2 µM. Addition of cytokinins to the basal regeneration medium enhances subsequent shoot formation. The cultures are incubated under the standard growth conditions (example 1) for 1-3 months.

### Stage 3: Rooting

One- to three-cm tall shoots are transferred to standard in vitro culture containers and preferably to 150-ml glass test tubes filled with 18 ml to 25 ml of a rooting medium consisting of the basal regeneration medium supplemented with (i) 1 µM to 3 µM of auxin, preferably IAA and preferably at a concentration of 1 µM, and (ii) 1 µM to 3 µM of cytokinin, preferably BAP and preferably at a concentration of 1 µM. Root formation usually takes place within 1-2 months of culture under the standard growth conditions (example 1). More than 90% of plants of 10-15 cm height regenerated with this procedure survive the transfer to full ground.

The above described regeneration protocol is superior to the one described by Dhed'a et al. (1991) as it eliminates one step and avoids the use of liquid media. In addition, this protocol results in a high frequency of plant regeneration which falls between 7·10³ to 5·10⁴ plants per ml of settled cell volume of cells. The frequency of somaclonal variation during vegetative development of plants regenerated from scalp-derived embryogenic cultures and cell suspensions does not exceed 2% (Table 2) which compares favourably with the control plant populations derived from classical micro-propagation.

Because of their high regeneration potential and the low rate of somaclonal variation, the scalp-derived embryogenic cell suspensions may also be applied to large-scale clonal propagation in banana. This could be achieved by mass cultivation in bioreactors. The large number of embryos obtained through somatic embryogenesis can also be used for the production of artificial seeds (Redenbaugh et al., 1986) by encapsulating them in alginate or agar beads.

**Table 2**

| Percentage of off-type plants regenerated from embryos directly derived from embryogenic cultures (EC) or from embryogenic cell suspenions (ECS) of three banana cultivars. Control plants are regenerated from shoot-tip cultures (STC). Evaluations are performed in the vegetative development phase. | | | | |
|---|---|---|---|---|
| Cultivar | Origin | No. of plants | No. of off-types | % of off-types |
| Williams | STC | 20 | 3 | 15.0 |
| | ECS | 110 | 2 | 1.8 |
| Agbagba/Harton | ECS | 105 | 0 | 0.0 |
| Three Hand | STC | 20 | 0 | 0.0 |
| Planty | EC | 20 | 0 | 0.0 |
| | ECS | 50 | 0 | 0.0 |

### EXAMPLE 5

### Isolation of protoplasts from embryogenic banana cell suspensions and subsequent regeneration of plants through somatic embryogenesis

For cereals, embryogenic cell suspensions have been the only source of regenerable protoplasts. Similarly, embryogenic cell suspensions of bananas proved to be suitable for the isolation of protoplast with capacity for plant regeneration (Panis et al., 1993). These regenerable protoplasts are suitable for genetic transformation by electroporation (example 6) and may also be applied to somatic hybridization.

### Protoplast isolation.

One to three weeks and preferably two weeks after their last subculture, embryogenic cells of but not restricted to the cooking banana cultivar Bluggoe are concentrated to a packed cell volume (PCV) between 10% and 30% and preferably 20% (v/v). One ml of this concentrated suspension is subsequently transferred to 6 ml of enzyme solution containing cellulase Onozuka R-10 from Trichoderma viridae, macerozyme R-10 from Rhizopus spp., and pectinase 5S from Aspergillus niger alone or in combinations at a concentration of 0.1% to 2.0%. The enzyme solution also contains 7 mM CaCl₂·2H₂O, 0.7 mM NaH₂P0₄·2H₂O, 3 mM 2-N-morpholinoethane sulphonic acid and 10% (w/v) mannitol. The pH is adjusted to 5.8 and the solution filter-sterilized. Cells are incubated at 40 rpm on a rotary shaker in the dark for periods ranging between 4 h and 24 h. Optimal protoplast yields using a 2-week-old suspension are
and after an enzymatic digestion of 24 h reach as many as 70 million protoplasts per ml PCV of cells.

### Protoplast purification.

After the enzyme treatment, the protoplast-containing suspension still contains undigested cell clumps and cell debris. Therefore, the suspension is washed by centrifugation at 66 g for 5 min. with 10 ml MS medium devoid of plant growth regulators but supplemented with 10% (w/v) mannitol. Then, protoplasts are purified either by flotation on 20% to 25% (w/v) sucrose or sieving through a 100-µm and subsequently through a 25-µm filter. Purified protoplasts are counted before and after purification to determine purification efficiencies. Cell wall degradation and viability are visualized with a UV fluorescent microscope using Calcofluor white and fluorescein diacetate, respectively. The purification efficiency with the flotation method varies between 15% and 35%, while the sieving method results in only 0-26% loss of protoplasts after purification.

### Protoplast culture.

Protoplasts are resuspended at densities ranging between 10⁵ and 10⁶ protoplasts per ml in half-strength Murashige and Skoog (MS) medium containing 5 µM 2,4-D and 10% (w/v) mannitol. Three regeneration methods are successfully applied.

### 1/ Culture on solid medium:

One ml of the diluted suspension is placed on a half-strength MS medium containing 5 µM 2,4-D, 5% (w/v) mannitol, and 0.8% (w/v) agarose or 0.2% (w/v) Gelrite (Phytagel TM, Sigma), with or without covering it with a black gridded 0.8-µm filter (Millipore, n°AABG04700).

### 2/ Culture with feeder layers:

This method is based on the principle developed by Rhodes et al. (1988). In a Petri dish with a diameter of 90 mm, 20 ml of half-strength MS medium supplemented with 5 µM 2,4-D, 5% (w/v) mannitol, and 0.2% (w/v) Gelrite is poured. On this layer, 2 ml of the nurse culture is placed which is obtained by mixing 1 ml of a 1-week-old embryogenic cell suspension concentrated to 5% (v/v) PCV with 1 ml MS medium supplemented with 5 µM 2,4-D, 10% (w/v) mannitol, 200 mg/l myo-inositol, and 1.6% (w/v) agarose. On top of the feeder layer, a black Millipore filter (see above) is placed, and onto the filter 1 ml of protoplasts is gently poured.

### 3/ Culture on preconditioned medium:

This method resembles the previous one. However, instead of 1 ml of embryogenic cell suspension, 1 ml of a preconditioned medium is used. The preconditioned medium is prepared by separating the medium from a 1-week-old cell culture with a Whatman filter followed by filter-sterilization.

Two basic strategies are applicable for achieving sustained division (more than 50%) in banana protoplasts, i.e. the use of feeder cells or plating protoplasts at very high (10⁶ protoplasts per ml) concentration. The latter is successful with each of the three culture methods applied. Five weeks after inoculation, equally sized microcolonies are formed containing about 100 cells with embryogenic characteristics similar to embryogenic cell suspensions. These microcolonies are transferred to a standard MS medium devoid of plant growth regulators where they develop into somatic embryos. Rooted plants are regenerated 4 months after protoplast isolation.

### EXAMPLE 6

### Cryopreservation of proliferating meristems of banana

Cryopreservation of proliferating meristems serves two purposes. Firstly, the starting material for induction of embryogenic cell cultures can be stored without the risk of microbial contamination. Secondly, transgenic shoot cultures can be stored safely on a long term. Recently, a cryopreservation method for proliferating 'cauliflower-like' meristems has been developed (Panis et al., 1996) which consists of a meristem preculture step on media containing high sugar concentrations followed by rapid freezing. Post-thaw culture proceeds on semi-solid regeneration medium. In this example, we describe an improved cryopreservation protocol using liquid regeneration media which results in significantly higher post-thaw survival rates.

### Starting material.

For meristem cryopreservation, 'cauliflower-like' proliferating meristem cultures are required (see example 1).

### Preculture.

From proliferating meristem cultures, white meristematic clumps of about 4 mm diameter, each containing at least four apical domes are excised and transferred onto a preculture medium. This medium consists of semi-solid MS medium supplemented with 10 µM BAP and 1 µM IAA and enriched with sucrose, glucose or fructose or a combination of these sugars to a final concentration of 0.3 M to 0.6 M, preferably sucrose and preferably at a concentration of 0.4 M. These cultures are kept for about 1 week to about 4 weeks and preferably for about 2 weeks under conditions identical to those for normal meristem culture (example 1).

### Cryopreservation and thawing.

Small clumps of 5-15 mg, containing 3 to 6 meristematic domes are excised from the precultured buds. Brown tissues are removed and only the most healthy part as indicated by a white-yellowish color is selected. These clumps are transferred to 2-ml sterile cryotubes and directly plunged into liquid nitrogen. Each cryotube contains 7 to 10 clumps. After storage in liquid nitrogen, the frozen cryotubes are thawed by stirring for 1.5 min. in a water bath of 40 °C.

### Post-thaw recovery.

Thawed explants are transferred to 100-ml Erlenmeyer flasks containing 30 ml liquid MS medium supplemented with 10 µM BAP, 1 µM IAA and 3% (w/v) sucrose, and placed on a rotary shaker at about 40 rpm to about 90 rpm and preferably at about 70 rpm. In the first week following thawing, the cultures are placed for 1 week in the dark after which they are placed in the culture room under standard growth conditions. Six weeks after freezing, regrowth is determined under a binocular microscope. The regeneration frequencies of a number of banana cultivars belonging to different genomic groups are listed in Table 3. Recovering clumps are transferred to test tubes containing a semi-solid MS medium supplemented with 1 µM BAP, 1 µM IAA and 3% (w/v) sucrose. As soon as rooted plants reach the top of the test tube, they are transplanted in the soil.

**Table 3**

| Regeneration frequencies after cryopreservation of proliferating meristems of banana cultivars belonging to different genomic groups | | |
|---|---|---|
| **Group** | **Cultivar** | **Regeneration frequency (%)** |
| Cavendish (AAA) | Grande Naine | 19.2 |
| dessert bananas | Williams | 12.5 |
| | Cavendish 901 | 7.4 |
| AAB dessert bananas | Prata, clone 1 | 53.8 |
| | Prata, clone 2 | 10.0 |
| | Lady Finger | 33.3 |
| AAB plantains | Dominico Harton | 42.5 |
| | Agbagba/Harton | 12.3 |
| | Three Hand Planty | 30.0 |
| ABB cooking bananas | Bluggoe, clone 1 | 33.3 |
| | Bluggoe, clone 2 | 49.1 |
| | Saba | 68.9 |
| AA edible cultivar | Guyod | 34.4 |
| AB edible cultivars | Kamaramasenge | 20.5 |
| | Kisubi | 41.3 |
| BB wild diploid | M. balbisiana Tani | 29.9 |

### EXAMPLE 7

### Cryopreservation of banana embryogenic cells

During prolonged cell suspension culture there is always a risk of loosing this material due to microbial contamination, loss of regeneration capacity and somaclonal variation. It is therefore important to conserve this valuable material in a secure way. At ultra-low temperatures (cryopreservation), all metabolical, physical and chemical processes are arrested. As such, the material can be safely stored for unlimited periods. Panis et al. (1990) described a freezing method for embryogenic cell suspensions of the cooking banana cultivar Bluggoe. However, generalization of this freezing method towards embryogenic cell suspensions of a large number of banana cultivars can only be obtained if high concentrations of sucrose are added to the cryoprotective medium. The present cryopreservation protocol is developed for established embryogenic cell cultures (example 3). However, scalp-derived embryogenic cultures (example 2) and globular somatic embryos (example 4) may also be subjected to cryopreservation.

### Cryoprotection.

An equal volume of sterile MS medium containing dimethylsulfoxide (DMSO) and sucrose is gradually added to a concentrated cell suspension during one hour until a final settled cell volume of 5% to 15% (v/v), a DMSO concentration of 5% to 15% (v/v) and a sucrose concentration of 0.087 M to 0.53 M is obtained. Best results are achieved using a settled cell volume of 10% (v/v), a DMSO concentration of 7.5% (v/v) and a sucrose concentration of 0.3 M.

### Freezing, storage and thawing.

One and a half ml aliquots of the cryoprotected suspensions are transferred to 2-ml cryotubes and placed in a stirred methanol bath. This methanol bath cools at a rate of 1°C/min. As soon as about -5°C to about -10°C and preferably about -7.5°C is reached, the cryotubes are immersed for 3 seconds in liquid nitrogen to initiate ice crystallization. Then, they are further cooled to -40°C. After 30 min. at -40°C, the cryotubes are immersed for storage in liquid nitrogen. Subsequently, cryotubes are rapidly thawed in a beaker filled with sterile water of 40°C for about 1.5 min. until most of the ice is melted.

### Recovery.

Half-ml aliquots taken from the frozen-thawed cryotubes are placed on sterile filter papers on semi-solid medium ZZ in 90 mm Petri dishes. For regeneration of somatic embryos, the frozen-thawed aliquot is dropped on a semi-solid MS medium supplemented with 1 µM BAP. During the first week after thawing Petri dishes are kept in the dark. Cell viability is determined by the fluorescein diacetate test whereby living cells show very bright fluorescence under UV illumination. Thawed cells placed on the semi-solid ZZ medium are also inoculated into liquid ZZ medium to form an embryogenic cell suspension comparable to the original material. The reinitiated cell suspensions are regenerated in culture tubes containing semi-solid basal regeneration medium following the protocol described in example 4.

This cryopreservation method is successful for embryogenic cell suspensions of several cultivars belonging to different genomic groups including but not restricted to the Cavendish group of dessert bananas, the cooking bananas, the plantains, and wild diploid species such as Musa balbisiana. Since these groups represent a significant range of genetic diversity in bananas it is assumed that this cryopreservation method offers wide if not universal applicability in banana.

### EXAMPLE 8

### Genetic transformation of banana protoplasts

### Cell suspensions and plasmids.

Embryogenic cell suspensions (ECSs) are maintained and subcultured weekly as described in example 3. The ECSs are cultured for at least 1 year prior to use in the experiments and consist of small clusters of isodiametric, cytoplasm-rich cells.

The plasmids used for transformation are propagated in the E. coli DH5α strain. Plasmid DNA prepared by alkaline lysis is purified on Qiagen columns (Diagen GmbH). DNA concentrations are determined by absorption measurements at 260 nm and by gel electrophoresis.

### Protoplast isolation.

Isolation and purification of protoplasts from embryogenic cell suspensions is done by strictly following the procedure outlined in example 5. Purified protoplasts are resuspended in electroporation buffer at a concentration of 1.25 x 10⁶ protoplasts per ml. The electroporation buffer is composed of 70 mM potassium aspartate, 5 mM calcium gluconate, 5 mM 2-N-morpholino-ethane sulphonic acid, and 0.55 M mannitol, the pH is adjusted to 5.8 (Tada et al., 1990). Protoplasts are counted by using a modified Neubauer haemocytometer. Complete removal of the cell wall is confirmed by Calcofluor white staining while the viability of freshly isolated or electroporated protoplasts is routinely assessed by staining either with fluorescein diacetate or Evans' Blue.

### Transformation procedure.

For transformation of protoplasts by electroporationm is described by Sági et al. (1994). A 800 µl aliquot containing 10⁶ protoplasts in electroporation buffer is placed into an electroporation cuvette with a 0.4 cm gap. After addition of plasmid DNA to a concentration of 60 µg/ml, cuvettes are stored on ice for 10 min., then electroporated with a 960 µF capacitor of a Bio-Rad Gene Pulser™ transfection apparatus or of an equivalent at a field strength of 800 V/cm. After electroporation, the cuvettes are placed on ice for 10 min. and then for 10 min. room temperature. Protoplasts are diluted with ZZ medium supplemented with 0.55 M mannitol to a concentration of 10⁵ protoplasts per ml and incubated in the dark at 24°C. The following controls are used: (i) samples electroporated with pUC19 plasmid DNA, (ii) samples electroporated without plasmid DNA, (iii) non-electroporated samples incubated with the plasmid DNA used for electroporation.

### β-glucuronidase expression assay.

For the histochemical in situ assays, protoplasts are collected 48 hours after electroporation, resuspended in 50 mM sodium phosphate buffer, pH 7.0 and incubated for periods ranging from overnight up to 10 days at 37°C in the presence of 1 mM X-Gluc (5-bromo-4-chloro-3-indolyl-β-D-glucuronide) as described by Jefferson (1987). Protoplast transformation frequency is assessed by counting blue-stained protoplasts and relating this to the total number of protoplasts. Two to four internal repeats are counted and averaged for each treatment in each experiment. On average 10⁵ protoplasts are observed for each repeat. The total number of treated protoplasts is determined independently for each repeat. Cultures of E. coli are used in parallel as positive controls for the GUS assay.

### EXAMPLE 9

### Genetic transformation of embryogenic cell suspensions of banana

### Plant material and culture conditions.

Embryogenic cell suspensions (ECSs) and proliferating meristems or scalps of various banana cultivars including but not restricted to the Cavendish dessert banana cultivar Williams (Musa spp., AAA group), and those described in example 3 are used for genetic transformation. These ECSs are cultured in ZZ medium (described in example 2), maintained on a rotary shaker as described in example 3, and subcultured at least once but preferably twice each two weeks. Cells are collected about 3 days to about 6 days after subculture and preferably preferably 4 days after subculture and used for genetic transformation. Proliferating meristems are subcultured on medium p4 as described in example 1 and directly used for transformation. Scalps are prepared for transformation before or during induction of somatic embryogenesis as outlined in example 2.

### Preparation of DNA for particle bombardment.

Plasmid DNA is isolated from the E. coli DH5α strain by standard large-scale purification methods using a combination of alkaline lysis and polyethylene glycol precipitation or Qiagen columns. Since we recognize that uniform and reproducible coating of the particles with DNA is a key factor for successful transformation, freshly prepared and coated particles are used for each bombardment. Coating of tungsten particles (Sylvania M-17) is performed as follows. About 2 mg to 4 mg of particles and preferably 3 mg of particles is mixed with about 3 µg DNA to about 6 µg DNA, and preferably 5 µg DNA (at a concentration of about 0.6 µg/µl to about 1.2 µg/µl but preferably at 1.0 µg/µl) and while vigorously vortexing an equal volume of 1 M of calcium nitrate (between pH 4.0 and pH 10.0 but preferably at about pH 5.0) is added, then further vortexed for about 2 min. to about 4 min., and preferably for 3 min. Then, the coated particles are washed with 70% and 100% (v/v) ethanol. Finally, particles coated with DNA are resuspended in 50 µl of 100% (v/v) ethanol and pipetted (8 µl/shot) onto about 100 to about 1000 µm plastic or stainless steel meshes to allow for drying (between about 1 min. to about 5 min., preferably for about 2 min.) before particle bombardment.

### Particle bombardment of banana embryogenic cell suspensions.

About 20 µl to about 200 µl, and preferably 50-100 µl of suspension cells at a settled cell volume of about 20% to about 40% and preferably about 30% (v/v), equal to approx. 25-60 mg of fresh weight, are collected on an about 50 µm to about 200 µm, and preferably 100 µm plastic preferably nylon mesh with a diameter of about 10 to about 20 mm, and preferably 12 mm. Particle bombardment is carried out with a home-made helium-driven particle gun constructed according to Sági et al. (1995) under the following standard conditions: (i) target distance, about 8 cm to about 14 cm, preferably about 12 cm; (ii) helium pressure, about 6 bar to about 10 bar, preferably about 8 bar; (iii) vacuum, about -0.7 bar to about -0.9 bar, and preferably about -0.8 bar; (iv) one shots to three shots per target but preferably one shot per target, and (v) with or without an about 4 hours to about an overnight osmotic pretreatment on ZZ medium containing 0.2 M sorbitol plus 0.2 M mannitol.

### Determination of critical concentrations of selective agents.

Growth kinetics are determined for different ECSs by measuring the fresh weight increase of equal amount of embryogenic cells which are plated on selective ZZ media containing the following concentrations (mg/l) of the selective agents kanamycin, geneticin and hygromycin: 5, 10, 20, 50, 75, and 100. Measurements are carried out at two time points, two and four weeks of culture after the treatment started. All measurements are carried out in at least two replicates with two independent experiments. Selective agents and concentrations are considered to be optimal when minimal or no cell growth is observed after the two or four weeks of culture. Viability of killed or surviving cells is also confirmed by standard staining tests like those with fluorescein diacetate or triphenyltetrazolium chloride.

### Recovery and regeneration of transgenic banana plants.

Following particle bombardment, ECSs are cultivated on a solid ZZ medium (described in example 2) without selection for about 7 days to about 14 days, and preferably for about 10 days and then placed on solid or in liquid selective ZZ medium as described in example 3 but supplemented with about 25 mg/l to about 75 mg/l, and preferably 50 mg/l geneticin or hygromycin and subcultured each 2 weeks. After approx. 2-3 months of culture, actively growing cell aggregates are selected and individually subcultured on solid proliferation medium for 1-2 months with or without selection, then transferred onto non-selective or selective proliferation and regeneration medium as described in example 4.

### β-Glucuronidase (GUS) assays.

Histochemical and fluorometric GUS assays for transient and stable expression in transformed cells, and plant tissues are performed under standard assay conditions (Jefferson, 1987).

### DNA isolation, PCR and Southern analysis.

Genomic DNA is extracted for PCR and Southern analysis using a standard CTAB extraction method. A 50 µl PCR reaction mix contains the primers (1 µM final concentration each), Taq polymerase (1.0 U), 125 µM of each dNTP, 1 x PCR reaction buffer and approximately 50 ng DNA. Reactions are overlaid with 50 µl of mineral oil. PCR conditions are 94 °C initial melting for 3 min. followed by 30 cycles of 94 °C/1 min., 60 °C/1 min., 72 °C/2 min., with a 72 °C/7 min. final extension. PCR products and total genomic DNA are separated in 1.2% and 0.8% agarose gel, respectively, and blotted onto nylon membranes. Digoxigenin-labelled probes are prepared by PCR as described above, and used for hybridization according to standard protocols.

### FIGURE LEGENDS

Figure 1:
   A. Proliferating meristem culture of the cooking banana cultivar Bluggoe, 3 weeks after subculture.
   B. 'Cauliflower-like' aspect of a highly proliferating shoot-tip culture of the dessert banana cultivar Gros Michel, three weeks after subculture.
   C. High quality scalp of the cultivar Bluggoe derived from a meristem culture with 'cauliflower-like' aspect.
   D. Embryogenic culture of plantain cultivar Three Hand Planty 6 weeks after scalp induction. (bar = 1 mm)
Figure 2:
   A. Embryogenic cell aggregates in a cell suspension of the Cavendish banana cultivar Williams (bar = 10 µm).
   B. Logarithmic growth curve of the same cell suspension expressed in settled cell volume percentage (SCV%).

### REFERENCES

1. Banerjee N, De Langhe E (1985) A tissue culture technique for rapid clonal propagation and storage under minimal growth conditions of Musa (Banana and plantain). Plant Cell Reports 4: 351-354
2. Côte FX, Domergue R, Monmarson S, Schwendiman J, Teisson C, Escalant JV (1996) Embryogenic cell suspensions from the male flower of Musa AAA cv. Grand nain. Physiol Plantarum 97: 285-290
3. Cronauer-Mitra SS, Krikorian AD (1988) Plant regeneration via somatic embryogenesis in the seeded diploid banana Musa ornata Roxb. Plant Cell Reports 7: 23-25
4. Dhed'a D (1992) Culture de suspensions cellulaires et régénération en plantules par embryogénèse somatique chez le bananier et le bananier plantain (Musa spp.). PhD thesis, Katholieke Universiteit Leuven, 167 pp
5. Dhed'a D, Dumortier F, Panis B, Vuylsteke D, De Langhe E (1991) Plant regeneration in cell suspension cultures of the cooking banana cv. 'Bluggoe' (Musa spp. ABB group). Fruits 46: 125-135
6. Escalant JV, Teisson C (1989) Somatic embryogenesis and plants from immature zygotic embryos of the species Musa acuminata and Musa balbisiana. Plant Cell Reports 7: 665-668
7. Escalant J-V, Teisson C, Cote F (1994) Amplified somatic embryogenesis from male flowers of triploid banana and plantain cultivars (Musa spp.). In Vitro Cell Dev Biol 30P: 181-186
8. Jefferson RA (1987) Assaying chimeric genes in plants: the GUS fusion system. Plant Mol Biol Rep 5: 387-405
9. Marroquin CG, Paduscheck C, Escalant JV, Teisson C (1993) Somatic embryogenesis and plant regeneration through cell suspensions in Musa acuminata. In Vitro Cell Dev Biol 29P: 43-46
10. Murashige T, Skoog F (1962) A revised medium for rapid growth and bioassays with tobacco tissue cultures. Physiol Plantarum 15: 473-497
11. Novak FJ, Afza R, Van Duren M, Perea-Dallos M, Conger BV, Tang XL (1989) Somatic embryogenesis and plant regeneration in suspension cultures of dessert (AA and AAA) and cooking (ABB) bananas (Musa spp.). Bio/Technology 7: 154-159
12. Panis BJ, Withers LA, De Langhe EAL (1990) Cryopreservation of Musa suspension cultures and subsequent regeneration of plants. Cryo-Letters 11: 337-350
13. Panis B, Van Wauwe A, Swennen R (1993) Plant regeneration through direct somatic embryogenesis from protoplasts of banana (Musa spp.). Plant Cell Reports 12: 403-407
14. Panis B, Totté N, Van Nimmen K, Withers LA, Swennen R (1996) Cryopreservation of banana (Musa spp.) meristem cultures after preculture on sucrose. Plant Science 121: 95-106
15. Redenbaugh K, Paasch B, Nichol J, Kossler M, Viss P, Walker K (1986) Somatic seeds: encapsulation of asexual embryos. Bio/Technology 4: 797-801
16. Rhodes CA, Lowe KS, Ruby KL (1988) Plant regeneration from protoplasts isolated from embryogenic maize cell cultures. Bio/Technology 6: 56-60
17. Sági L, Remy S, Panis B, Swennen R, Volckaert G (1994) Transient gene expression in electroporated banana (Musa spp., cv. 'Bluggoe', ABB group) protoplasts isolated from regenerable embryogenic cell suspensions. Plant Cell Reports 13: 262-266
18. Sági L, Panis B, Remy S, Schoofs H, De Smet K, Swennen R, Cammue BPA (1995) Genetic transformation of banana and plantain (Musa spp.) via particle bombardment. Bio/Technology 13: 481-485
19. Schenk RU, Hildebrandt AC (1972) Medium and techniques for induction and growth of monocotyledonous and dicotyedonous plant cell cultures. Can J Bot 50: 199-204
20. Tada Y, Sakamoto M, Fukimura T (1990) Efficient gene introduction into rice by electroporation and analysis of transgenic plants: use of electroporation buffer lacking chloride ions. Theor Appl Genet 80: 475-480

## Claims

1. Plant regeneration method, comprising the provision of a starting material for regeneration and the regeneration of plants from the starting material, characterized in that, the provision of a starting material comprises proliferating in vitro meristems in the presence of cytokinins in a concentration of at least 50 µM, preferably at least 100µM to obtain cells in suspension.

2. Plant regeneration method of claim 1, wherein the step of the regeneration of plants is initiated by induction of somatic embryogenesis of cells in culture on semi-solid medium.

3. Plant regeneration method of claim 2, wherein the starting material for the induction of somatic embryogenesis is provided by any method for the preparation of cell cultures other than the one claimed in claim 1.

4. Plant regeneration method of claims 1-3, further comprising:
a previous or intermediate cryopreservation step either prior to or after the provision of the cell suspension, which cryopreservation step comprises in the case of cell suspensions the addition of increased amounts of sucrose to the material to be cryopreserved at a concentration of at least 0.3 M to obtain a cryoprotected material and slow freezing of the material; and
in the case of proliferating meristems, a post-thaw regeneration step, by which the cryopreserved material is regenerated on liquid regeneration medium.

5. Plant regeneration method as claimed in claims 1-4, further comprising a genetic transformation step of the cells in any of the steps before plant regeneration.

6. Plant regeneration method as claimed in claim 5, wherein the genetic transformation is performed on protoplasts prepared from the cells in suspension.

7. Plant regeneration method as claimed in claims 1-6, wherein the plant material originates from banana or plantain.

8. Plant regeneration method as claimed in claim 7, wherein the banana or plantain plants are selected from the following cultivars: Williams, Grande Naine, Cavendish 901, Gros Michel, Highgate, Igitsiri, Nakitengwa, Prata, Lady Finger, Silk, Agbagba/Harton, Three Hand Planty, Bise Egome-1, Dominico Harton, Bluggoe, Cardaba, Saba, Pisang Awak, Guyod, Kamaramasenge, Kisubi, M. balbisiana Tani.

9. Proliferation medium (p4 medium), comprising MS medium, which is supplemented with (i) 0.5 µM to 2.5 µM of auxin, preferably IAA and preferably at a concentration of 1 µM, (ii) 50 µM to 250 µM of cytokinin, preferably benzyladenin (BAP) and preferably at a concentration of 100 µM, (iii) 2% to 4% (w/v) of a sugar selected from the group sucrose, glucose, maltose, fructose or combinations thereof, preferably sucrose, at a preferable concentration of 3% (w/v), and solidified with 0.2% (w/v) of Gelrite.
